(19) **Europäisches Patentamt / European Patent Office / Office européen des brevets**

(11) **EP 4 483 789 A1**

(12) **EUROPEAN PATENT APPLICATION**
published in accordance with Art. 153(4) EPC

(43) Date of publication:
**01.01.2025 Bulletin 2025/01**

(21) Application number: **22928585.3**

(22) Date of filing: **22.02.2022**

(51) International Patent Classification (IPC):
**A61B 5/01** (2006.01)

(52) Cooperative Patent Classification (CPC):
**A61B 5/01**

(86) International application number:
**PCT/JP2022/007428**

(87) International publication number:
**WO 2023/162054 (31.08.2023 Gazette 2023/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Biodata Bank, Inc.**
**Tokyo 150-0031 (JP)**

(72) Inventors:
• **KODERA, Yuya**
  **Tokyo 150-0031 (JP)**
• **SHIOTSU, Takahiro**
  **Tokyo 150-0031 (JP)**
• **HIRAYAMA, Katsuhiro**
  **Tokyo 150-0031 (JP)**

(74) Representative: **Patentanwälte Bauer Vorberg Kayser**
**Partnerschaft mbB**
**Goltsteinstraße 87**
**50968 Köln (DE)**

(54) **INTERNAL TEMPERATURE MEASUREMENT DEVICE, INTERNAL TEMPERATURE MEASUREMENT METHOD, AND PROGRAM**

(57) To provide an internal temperature measurement device being manufactured at low costs, an internal temperature measurement method and a program. A deep body thermometer (100) comprises a measurement part (1) and a controller part (6). The measurement part (1) comprises a thermo-electric element (112) for measuring a heat flux amount HF from the internal position of subject as a subject to be measured and a temperature sensor (111) for measuring temperature T of a face of the thermo-electric element 112) at a side of the subject to be measured. The measurement part (1) outputs a heat flux amount HF1 measured by the thermo-electric element (112), a temperature T1 measured by the temperature sensor (111) at the heat flux amount HF1, a heat flux amount HF2 different from the heat flux amount HF 1 measured by the thermo-electric element (112) and a temperature T2 measured by the temperature sensor (111) at the heat flux amount HF2. The controller part (6) obtains an internal temperature of the subject to be measured based on the heat flux amount HF1, the temperature T1, the heat flux amount HF2 and the temperature T2 input from the measurement part (1).

Fig. 1

EP 4 483 789 A1

**Description**

Technical Field

The present invention relates to an internal temperature measurement device, an internal temperature measurement method and a program, and more particularly relates to an internal temperature measurement device being manufactured at low costs, an internal temperature measurement method and a program.

Background Art

As a device measuring a deep body temperature, a deep body thermometer, which measures the deep body temperature using two heat-flux sensors to which a temperature sensor (temperature measurement element) is attached at each of the upper and lower faces of heat resistance (heat insulating material) having relatively large areas, has been known (for example, refer to Patent Literature 1.) Now, description, claims, and entire drawings of Patent Literature 1 can be incorporated herein by reference.

Prior Art Literature

Patent Literature

Patent Literature 1: JP2007-212407

Summary of Invention

Problem to be Solved by Invention

However, a conventional deep body thermometer, since manufacturing requires a sandwich structure in which the heat flux sensor is sandwiched by two temperature sensors, has presented the problem that production costs become expensive.

The present invention is completed to solve the above problem and aims to provide an internal temperature measurement device being manufactured at low costs, an internal temperature measurement method and a program.

Means for Solving Problem

To achieve the above purpose, An internal temperature measurement device (100, 500, 700, 1000) of a first aspect of the present invention comprising: a thermo-electric element (112, 621, 622, 821) for measuring a heat flux from an internal position of an object to be measured; a temperature sensor (111, 611, 612) for measuring temperature of a face of the thermo-electric element (112, 621, 622, 821) at a side of the object to be measured; a measurement part (1, 41, 42, 51, 52, 71, 101) for outputting a first heat flux amount measured by the thermo-electric element (112, 621, 821), a first temperature measured by the temperature sensor (111, 611) at the first heat flux amount, a second heat flux amount different from the first heat flux amount measured by the thermo-electric element (112, 622, 821) and a second temperature measured by the temperature sensor (111, 612) at the second heat flux amount; and a controller part (6) for obtaining the internal temperature of the object to be measured based on the first heat flux amount, the first temperature, the second heat flux amount and the second temperature input from the measurement part (1, 41, 42, 51, 52, 71, 101).

In the above internal temperature measurement device (100, 700, 1000), the controller part (6) may obtain the first heat flux amount and the first temperature from the measurement part (1, 41, 42, 71); after changing temperature of a surface of the object to be measured by supplying electric power to the thermo-electric element (112, 822), obtains the second heat flux amount and the second temperature from the measurement part (1, 41, 42, 71); and may obtain an internal temperature of the object to be measured based on the first heat flux amount, the first temperature, the second heat flux amount and the second temperature.

In the above internal temperature measurement device (700), the measurement part (71) may further comprise a thermo-electric element (822) for heat source used as a heat source, and wherein the controller part (6) obtains the first heat flux amount measured by the thermo-electric element (821) and the first temperature measured by the temperature sensor (111), and after changing temperature of a surface of the object to be measured by supplying electric power to the thermo-electric element (822), may obtain the second heat flux amount measured by the thermo-electric element (821) and the second temperature measured from the temperature sensor (111).

In the above internal temperature measurement device (100, 700, 1000), the controller part (6), after supplying electric power to the thermo-electric element (112, 822) for a predetermined duration, may determine whether or not the heat flux amount input from the measurement part (1, 41, 42, 71) becomes constant, and when determined that the heat flux amount becomes constant, may obtain the heat flux amount and the temperature input from the measurement part (1, 41, 42, 71) as the second heat flux amount and the second temperature.

In the above internal temperature measurement device (1000), the temperature sensor (611, 612) may comprise a first temperature sensor (611) and a second temperature sensor (612); and wherein the measurement part (101) is arranged with disposing the second temperature sensor (612), the first temperature sensor (611) and the thermo-electric element (112) in approximately equal spacings; the controller part (6), when the temperature measured by the second temperature sensor (612) and the temperature measured by the first temperature sensor (611) are approximately same, may obtain temperature obtained by the first temperature sensor (611) as temperature of a face of the thermo-electric element (112) at a side of the object to be measured; and when the temperature measured by the second temperature sensor (612) and the temperature measured by the first temperature sensor (611) are not approximately same, may obtain temperature of a face of the thermo-electric element (112) at a side of the object to be measured from a difference between temperature measured by the second temperature sensor (612) and temperature measured by the first temperature sensor (611) and temperature measured by the first temperature sensor (611).

In the above internal temperature measurement device (500), the measurement part (51, 52) may comprise a first measurement part (51) and a second measurement part (52); and wherein the first measurement part (51) may comprise a first thermo-electric element (621) and a first temperature sensor (611) for measuring temperature of a face of the thermo-electric element (621) at a side of the object to be measured and outputs the first heat flux amount measured by the first thermo-electric element (621) and the first temperature measured by the first temperature sensor (611); and the second measurement part (52) may comprise a second thermo-electric element (622) having a heat resistance value different from the first thermo-electric element (621) and a second temperature sensor (612) for measuring temperature of a face of the thermo-electric element (622) at a side of the object to be measured; and may output the second heat flux amount measured by the second thermo-electric element (622) and the second temperature measured by the second temperature sensor (612).

An internal temperature measurement method of a second aspect of the present invention comprising: by a measurement part (1, 41, 42, 51, 52, 71, 101) comprising a thermo-electric element (112, 621, 622, 821) for measuring a heat flux amount from an internal position of an object to be measured and a temperature sensor (111, 611, 612) for measuring temperature of a face of the thermo-electric element (112, 621, 622, 821) at a side of the object to be measured; outputting a first heat flux amount measured by the thermo-electric element (112, 621, 821), a first temperature measured by the temperature sensor (111, 611) at the first heat flux amount, a second heat flux amount different from the first heat flux amount measured by the thermo-electric element (112, 622, 821) and a second temperature measured by the temperature sensor (111, 612) at the second heat flux amount; and obtaining an internal temperature of the object to be measured based on the first heat flux amount, the first temperature, the second heat flux amount and the second temperature input from the measurement part (1, 41, 42, 51, 52, 71, 101).

A program of a third aspect of the present invention makes a computer of an internal temperature measurement device (100, 500, 700, 1000) comprising a measurement part (1, 41, 42, 51, 52, 71, 101) comprising a thermo-electric element (112, 621, 622, 821) for measuring a heat flux amount from an internal position of an object to be measured and a temperature sensor (111, 611, 612) for measuring temperature of a face of the thermo-electric element (112, 621, 622, 821) at a side of the object to be measured and outputting a first heat flux amount measured by the thermo-electric element (112, 621, 821), a first temperature measured by the temperature sensor (111, 611) at the first heat flux amount, a second heat flux amount different from the first heat flux amount measured by the thermo-electric element (112, 622, 821) and a second temperature measured by the temperature sensor (111, 612) at the second heat flux amount to execute; obtaining an internal temperature of the object to be measured based on the first heat flux amount, the first temperature, the second heat flux amount and the second temperature input from the measurement part (1, 41, 42, 51, 52, 71, 101).

Advantageous Effect of Invention

According to the present invention, an internal temperature measurement device being manufactured at low costs, an internal temperature measurement method and a program can be provided.

Brief Description of Drawings

[Fig. 1] A block diagram illustrating an entire configuration of a deep body thermometer according to the present invention.
[Fig. 2] A cross-sectional view of a configuration example of measurement part according to the present invention.

[Fig. 3] A flowchart illustrating detail of a deep body temperature measurement processing.

[Fig. 4] (a) is a cross-sectional view of a configuration example according to the modified embodiment 1, and (b) is a cross-sectional view of a configuration example according to the modified embodiment 2.

[Fig. 5] A block diagram illustrating an entire configuration of a deep body thermometer according to a modified embodiment 3.

[Fig. 6] A cross-sectional view of a configuration example of a measurement part according to the modified embodiment 3.

[Fig. 7] A block diagram illustrating an entire configuration of a deep body thermometer according to a modified embodiment 4.

[Fig. 8] A cross-sectional view of a configuration example of a measurement part according to the modified embodiment 4.

[Fig. 9] A flowchart illustrating detail of a deep body temperature measurement processing according to a modified embodiment 4.

[Fig. 10] A block diagram illustrating an entire configuration of a deep body thermometer according to a modified embodiment 5.

[Fig. 11] A cross-sectional view of a configuration example of a measurement part according to the modified embodiment 5.

Embodiment for Practicing Invention

Now, embodiments for practicing the present invention will be described.

First, the configuration of deep body thermometer (internal temperature measurement device) of the present invention will be described with reference to drawings.

The deep body thermometer of the present invention (internal temperature measurement device) is attached on the body surface of central part such as the head or the body trunk of subject to be the object of measurement and obtains a heat flux amount from deep positions (internal position) such as brain or organs etc. so as to measure the deep body temperature (internal temperature), which is the internal body temperature at deep positions. Now, the measurement of internal temperature according to the present invention includes not only the measurement of internal temperature itself but also includes the estimation of internal temperature and the detection in the change of internal-temperature.

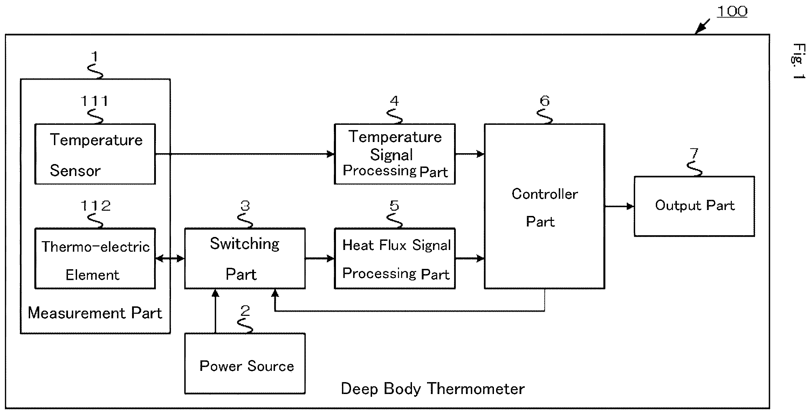

Fig. 1 shows a block diagram of the configuration example of deep body thermometer according to the present embodiment.

As shown in Fig. 1, the deep body thermometer 100 comprises a measurement part 1, a power source 2, a switching part 3, a temperature-signal processing part 4, a heat-flux signal processing part 5, a controller part 6, and an output part 7.

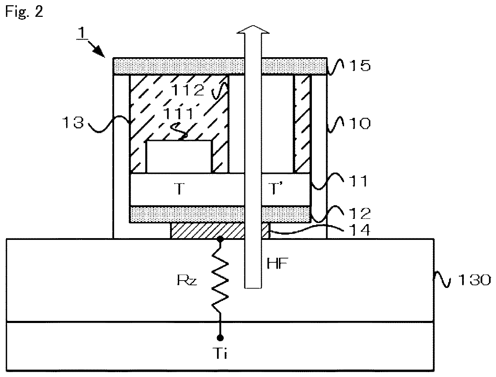

Fig. 2 shows a cross-sectional view illustrating the configuration example of measurement part according to the present embodiment.

As shown in Fig. 2, the measurement part 1 comprises, at the side of measuring face that contacts with the body surface of subject for measuring the deep body temperature and within a case 10 made from plastics, a substrate 11, a heat-diffusion layer 12, and a heatinsulation layer 13. Furthermore, the measurement part 1 comprises a heat collection plate 14 at the face of case 10 facing to the measuring surface side and a heat conduction sheet 15 at the face opposite thereto.

The substrate 11 is made of materials having insulation performance such as, for example, polyimide and the like, and in the present embodiment, is a flexible substrate (film substrate) having flexibility and being formed as the plane plate of 8mm × 10mm. Now, the substrate 11 should not be limited to the deformable flexible substrate and may also be a deformable printed circuit board.

The substrate 11 is arranged with disposing a temperature sensor 111 and a thermo-electric element 112 in close proximity on the face opposite to the measuring face such that the temperature T measured by the temperature sensor 111 and the temperature T' at the lower face (measuring face side) of thermo-electric element 112 becomes approximately equal. In this regard, the detail thereof is described in International Publication No. 2020/184511. Here, description, claims, and drawings of International Publication No. 2020/184511 are entirely incorporated herein by reference.

The temperature sensor 111 is configured from, for example, thermistors such as a NTC (Negative Temperature Coefficient) thermistor of which resistance value changes depending on the temperature. The temperature sensor 111 measures the temperature T of the subject-body surface. According to the present embodiment, from the point of view for improving responsibility, a heat capacity is low as low as possible such that the chip NTC thermistor is used as the temperature sensor 111. The temperature sensor 111 is electrically connected with the controller part 6 via. printed wirings not shown and outputs electric signals (voltage value) indicating the measured temperature T via. printed wirings not shown.

The thermo-electric element 112 is configured from for example, a Peltier element etc. The thermo-electric element 112 functions as a heat-flux sensor for obtaining the heat flux amount HF from deep positions such as subject's brain and organs etc. The thermo-electric element 112 is electrically connected with the controller part 6 via. printed wirings not shown and outputs the electric signals (voltage value) indicating the measured heat flux amount HF via. printed wirings not shown. Furthermore, the thermo-electric element 112 allows to flow direct currents when the electric power is supplied from the power source 2 to heat or cool the body surface of subject (by changing the temperature of object's surface to be measured) and also functions as a heat source for changing the heat flux amount HF from the deep positions.

The heat-diffusion layer 12 is configured by carbon heat conduction sheets and the like for attaining efficient heat conduction such as graphite sheets or carbon sheets etc. having extremely high heat conductivity (for example, 1350 [W/mK] and the like). Now, the heat diffusion layer 12 may be configured by metal thin films such as aluminum sheets or copper sheets. The heat diffusion layer 12 is disposed at the face of measuring surface side of substrate 11; diffuses the heat entered from a heat collection plate 14; and allows heat conduction to the direction horizontal to the measuring face such that temperature distribution within the face at the measuring surface of substrate 11 makes even. Thereby, the heat diffusion layer 12 can make the temperature T measured by the temperature sensor 111 and the temperature T' at the lower face (measuring face side) of thermo-electric element 112 approximately equal. As the result, the temperature sensor 111 can measure the temperature of subject side face (lower surface) of thermo-electric element 112.

The heat insulation layer 13 is configured from materials having heat insulating performance, for example, including an air layer not allowing air convection, polyimide, polyethylene foam and polyurethane foam and the like and is filled in the case 10 so as to cover the temperature sensor 111 and the thermo-electric element 112. The heat insulation layer 13 thermally insulates the temperature sensor 111 and the thermo-electric element 112 such that the heat becomes hard to escape to the direction parallel the measuring face while improving the measurement precisions of temperature sensor 111 and thermo-electric element 112.

The heat collection plate 14 is configured from metal plates etc., for example, from pure copper etc. which have larger heat conductivity when compared with the subject. The heat collection plate 14 collects the heat on the subject's body surface and transfers to the temperature sensor 111 and to the thermo-electric element 112 via. the heat diffusion layer 12 to make it possible that the temperature sensor 111 and the thermo-electric element 112 can each measure the temperature T and the heat flux amount HF of subject's body surface over the case 10 made from plastics.

The heat conduction sheet 15 is configured from metal thin films etc., for example aluminum sheets or copper foils having good heat conduction performance (for example, 3-4[W/mK] and the like.).

As described above, the measurement part 1 is arranged with equipping the heat collection plate 14 and the heat conduction sheet 15 so as to generate the temperature difference between the face at the measuring face side of thermo-electric element 112 and the face at the opposite side thereto while allowing to promote heat transfer to the direction perpendicular the measuring face.

The power source 2 shown in Fig. 1 is configured by batteries etc. for example, a primary battery or a secondary battery and the like for general purpose and supplies the electric power (for example, to the extent of not more than 0.5 W) to the thermo-electric element 112.

The switching part 3 is configured from, for example, a general-purpose switching circuit etc. The switching part 3 switches the motion (mode) of thermo-electric element 112 under the control of controller 6. Particularly, the switching part 3, when the thermo-electric element 112 is used as a heat flux sensor, electrically connects the thermo-electric elements 112 with the heat-flux signal processing part 5. Contradictory to the above, the switching part 3, when the thermo-electric element 112 is used as the heat source, electrically connects the thermo-electric element 112 with the power source 2.

The temperature-signal processing part 4 and heat-flux signal processing part 5 are configured from general-purpose amplifiers and general-purpose A/D (Analog-to-digital) converters (ADC) etc. The temperature-signal processing part 4 amplifies analog electric signals input from the temperature sensor 111; then converts to digital electric signals; and outputs them. The heat-flux signal processing part 5 amplifies analog electric signals input from the thermo-electric element 112; then converts to digital electric signals; and outputs them.

The controller part 6 is configured from, for example, MCU (Micro Control Unit), ROM (Read Only Memory) and RAM (Random Access Memory) etc. MCU uses RAM as a working memory and executes adequately various programs etc. stored in ROM to control the various motions of deep body thermometer 100. According to the present embodiment, the controller part 6 measures the deep body temperature of subject based on the temperature T indicated by the electric signals input from the temperature-signal processing part 4 and the heat flux amount HF indicated by the electric signals input from the heat-flux signal processing part 5. Here, since the temperature T measured by the temperature sensor 111 and the temperature T' at the lower face (measuring face side) of thermo-electric element 112 are approximately equal, the temperature T indicated by the electric signals input from the temperature-signal processing part 4 is treated as to indicate the temperature T' at the lower face (measuring face side) of thermo-electric element 112.

Particularly, the controller part 6 outputs a first control signal for instructing electric connection between the thermo-electric element 112 and the heat-flux signal processing part 5 to the switching part 3 to make the thermo-electric element 112 function as the heat flux sensor. Next, the controller part 6 obtains the temperature T ($\fallingdotseq$T') indicated by the electric signal input from the temperature-signal processing part 4 and the heat flus amount HF indicated by the electric signal input from the heat-flux signal processing part 5 as the temperature T1 and the heat flux HF1 of subject at the normal condition before heating or cooling the body surface of subj ect. With respect to the temperature T1 and heat flux amount HF 1 obtained under the normal condition, the following relation (1) is satisfied.

$$(Ti-T1)/Rz=HF1 \qquad\qquad (1)$$

Here, Ti is the deep body temperature, and Rz is the thermal resistance value of subject's subcutaneous tissue 130, which are unknown values, respectively.

Subsequently, the controller part 6 outputs to the switching part 3 a second control signal for instructing electric connection between the thermo-electric element 112 and the power source 2 to make the thermo-electric element 112 function as the heat source. In other word, the controller part 6 changes the temperature of subject's surface by supplying the electric power to the thermo-electric element 112. After passing a predetermined duration from functioning the thermo-electric element 112 as the heat source (for example, several-ten seconds to one minute), the controller part 6 obtains the temperature T (≒T') indicated by the electric signal input from the temperature-signal processing part 4 and the heat flux amount HF indicated by the electric signal input from the heat-flux signal processing part 5. Furthermore, the controller part 6 determines whether or not the obtained heat flux amount HF becomes constant, and if not becoming constant, obtains the temperature T and the heat flux amount HF again after the predetermined duration has passed. Contradictory to the above, the controller part 6, when the obtained heat flux amount HF becomes constant, obtains the temperature T (≒T') indicated by the electric signal input from the temperature-signal processing part 4 and the heat flus amount HF indicated by the electric signal input from the heat-flux signal processing part 5 as the temperature T2 and the heat flux HF2 of subject at the thermal equilibrium condition. With respect to the temperature T2 and the heat flux amount HF2 obtained under the thermal equilibrium condition, the following relation (2) is satisfied.

$$(Ti-T2)/Rz=HF2 \qquad\qquad (2)$$

The unknown values in the above equations (1) and (2) are only the thermal resistance value Rz of subcutaneous tissue 130 and the deep body temperature Ti. Thus, by solving a simultaneous equation about the thermal resistance value Rz of subcutaneous tissue 130 and the deep body temperature Ti to eliminate the thermal resistance value Rz of subcutaneous tissue 130 from equations (1) and (2), which varies depending on positions or varies among individuals, the following equation (3) is obtained.
[Equation 3]

$$Ti = \frac{HF_2 T_1 - HF_1 T_2}{HF_2 - HF_1} \cdots (3)$$

Furthermore, the controller 6 obtains the deep body temperature of subject Ti by substituting into the equation (3) the temperature T1 and the heat flux amount HF1 obtained at the normal condition together with the temperature T2 and the heat flux amount HF2 obtained at the thermal equilibrium condition.

The controller part 6 displays the obtained deep body temperature on the display screen of output part 7 to acknowledge the deep body temperature Ti to the subject.

Here, the controller part 6 may determine whether or not the subject has the fear of thermic fever from the transition of deep body temperatures. Furthermore, the controller part 6, if a predetermined condition is satisfied such as the case where the deep body temperature exceeds a predetermined threshold value (dangerous value) or the case where the change in the deep body temperature exceeds a predetermined range, determines that there is the fear of thermic fever and may display on the display screen of output part 7; may generate alarm sounds from a speaker; and may light on or flash LED to acknowledge the subject for the fear of thermic fever.

The output part 7 can be configured from, for example, a liquid crystal display device etc. for general purpose. The output part 7 displays on the display screen the deep body temperature Ti which the controller part 6 has obtained. Here, the output part 7 is configured from a non-volatile memory etc. for general-purpose, for example, from a flash memory and the like and from one that stores the deep body temperature Ti that the controller part 6 has obtained. Furthermore, the output part 7 may be configured from, for example, a wireless transmission device etc. for general-purpose and may be one that the deep body temperature Ti obtained by the controller part 6 is transmitted to an outside server computer through a network such as INTERNET and the like. Furthermore, the output part 7 may be one that are combined with a liquid crystal display device for general-purpose, a non-volatile memory for general-purpose and a wireless transmission device for general-purpose.

Next, the deep body temperature measurement processing that is executed by the deep body thermometer 100 comprising the above configuration will be described with reference to drawings.

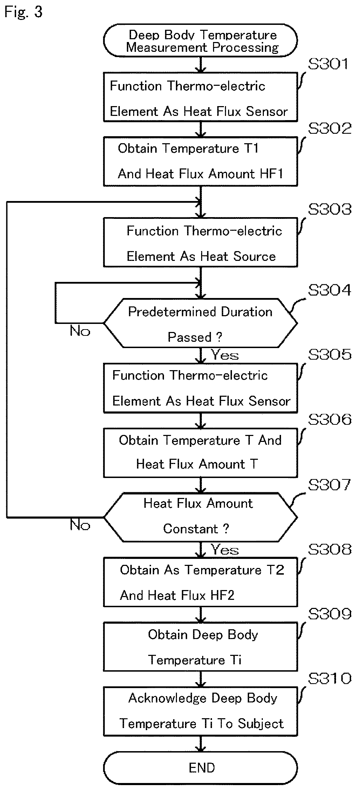

Fig. 3 is a flowchart illustrating the detail of deep body temperature measurement processing of the present embodiment.

In the deep body temperature measurement processing shown in Fig. 3, the controller part 6 of deep body thermometer 100 first outputs to the switching part 3 a first control signal for instructing the electric connection between the thermo-electric element 112 and the heat-flux signal processing part 5 to make the thermo-electric element 112 function as the heat flux sensor (Step S301).

Next, the controller part 6 obtains the temperature T ($\fallingdotseq$T') indicated by the electric signal input from the temperature-signal processing part 4 and the heat flus amount HF indicated by the electric signal input from the heat-flux signal processing part 5 as the temperature T1 and the heat flux HF1 of subject at the normal condition before heating or cooling the body surface of subject (Step S302).

Subsequently, the controller part 6 outputs to the switching part 3 a second control signal for instructing electric connection between the thermo-electric element 112 and the power source 2 to make the thermo-electric element 112 function as the heat source (Step S303).

Thereafter, the controller part 6 determines whether or not the predetermined duration has passed after the thermo-electric element 112 has been made to function as the heat source (Step S304), and if not having passed (Step S304; No), loops to wait.

Then, the controller part 6 outputs, when the predetermined duration has passed (Step S304; Yes), the first control signal to the switching part 3 and makes the thermo-electric element 112 function again as the heat flux sensor (Step S305).

Next, the controller part 6 obtains the temperature T ($\fallingdotseq$T') indicated by the electric signal input from the temperature-signal processing part 4 and the heat flus amount HF indicated by the electric signal input from the heat-flux signal processing part 5 (Step S306).

Furthermore, the controller 6 determines whether or not the heat flux amount HF obtained in Step S306 becomes constant (Step S307), and if not becoming constant (Step S307; No), returns to Step S303 to obtain the temperature T and the heat flux amount HF again after the predetermined duration has passed.

Then, the controller part 6, when the heat flux HF obtained in Step S306 becomes constant (Step S307; Yes), obtains the temperature T ($\fallingdotseq$T') indicated by the electric signal input from the temperature-signal processing part 4 and the heat flus amount HF indicated by the electric signal input from the heat-flux signal processing part 5 as the temperature T2 and the heat flux HF2 of subject at the thermal equilibrium condition (Step S308).

Subsequently, the controller part 6 obtains (measures) the deep body temperature of subject Ti by substituting into the equation (3) the temperature T1 and the heat flux amount HF1 obtained at the normal condition together with the temperature T2 and the heat flux amount HF2 obtained at the thermal equilibrium condition (Step S309).

Then, the controller part 6 displays the obtained deep body temperature Ti in Step S309 on the display screen of output part 7 so as to acknowledge the deep body temperature Ti to the subject (Step S310) and terminates the deep body temperature measurement processing.

As described above, the deep body thermometer (internal temperature measurement device) 100 of the present invention comprises the measurement part 1 and the controller part 6. The measurement part 1 comprises the thermo-electric element 112 for measuring the heat flux HF from the inside of subject as the object for the measurement and the temperature sensor 111 for measuring the temperature T at the subject' side face of thermo-electric element 112. The measurement part 1 outputs the heat flux amount HF 1 measured by the thermo-electric element 112, the temperature T1 measured by the temperature sensor 111 at the heat flux amount HF1, the heat flux amount HF2 different from the heat flux amount HF1 measured by the thermo-electric element 112, and the temperature T2 that the temperature sensor 111 has measured at the heat flux amount HF2. The controller part 6 obtains the deep body temperature Ti of subject based on the heat flux amount HF1, the temperature T1, the heat flux amount HF2, and the temperature T2 input from the measurement part 1.

Particularly, the controller part 6 obtains the heat flux amount HF1 and the temperature T1 from the measurement part 1. Next, the controller part 6 obtains, after changing the temperature of subject's surface by supplying the electric power to the thermo-electric element 112, the heat flux HF2 and the temperature T2 from the measurement part 1. In more detail, the controller part 6, after supplying the electric power for the predetermined duration to the thermo-electric element 112, determines whether or not the heat flux HF input from the measurement part 1 becomes constant. The controller part 6, if the heat flux amount HF is determined to become constant, obtains the heat flux amount HF and the temperature T input from the measurement part 1 as the heat flux amount HF2 and the temperature T2. Then, the controller part 6 obtains the deep body temperature Ti of subject based on the heat flux amount HF1, the temperature T1, the heat flux amount HF2

and the temperature T2.

As described above, the deep body thermometer 100, without providing the heat flux sensor having high-cost sandwich structure, can realize the approximately same function as the heat flux sensor having the sandwich structure by the temperature sensor 111 and the thermo-electric element 112 such that the deep body thermometer 100 can be manufactured in lower cost than conventional one.

Here, the present invention should not be limited to the above embodiment and may have various modifications and applications. Hereunder, the modified embodiments of above-described embodiment will be described, which are applicable to the present invention.

In the above embodiment, the description has been presented as one that the temperature sensor 111 and the heat flux sensor 112 are positioned in close proximity on the face opposite to the measuring face of substrate 11; however, the present invention should not be limited thereto.

[Modified Embodiment 1]

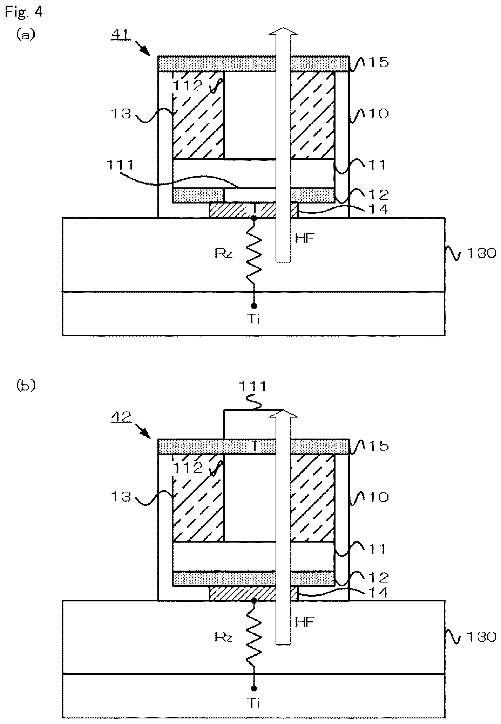

Fig. 4(a) shows the cross-sectional view of configuration example for the measurement part according to the modified embodiment 1.

As the measurement part 41 of modified embodiment 1 shown in Fig. 4 (a), the temperature sensor 111 is disposed at the lower face (face at the measuring side face) of thermo-electric element 112 and may be one that measures the temperature of lower face (face at the measuring side face) of thermo-electric element 112.

[Modified Embodiment 2]

Fig. 4 (b) shows the cross-sectional view of configuration example for the measurement part according to the modified embodiment 2.

Furthermore, as the measurement part 42 according to the modified embodiment 2 shown in Fig. 4 (b), the temperature sensor 111 may be disposed at the upper face (face at the side opposite to the measuring face) of heat conduction sheet 15 placed above the thermo-electric element 112.

In the above embodiment, the measurement part 1 has been described as one that the measurement part 1 comprises one temperature sensor 111 and one heat flux sensor 112, respectively; however, the present invention should not be limited thereto.

Hereinbelow, modified embodiments 3, 4 and 5 will be described. Here, configurations similar to the deep body thermometer 100 of the above embodiments are provided with the same signs and the description thereof will be omitted.

[Modified Embodiment 3]

First, the modified embodiment 3 of which measurement part comprises two temperature sensors and two thermo-electric elements, each of which has different thermal resistance, will be described.

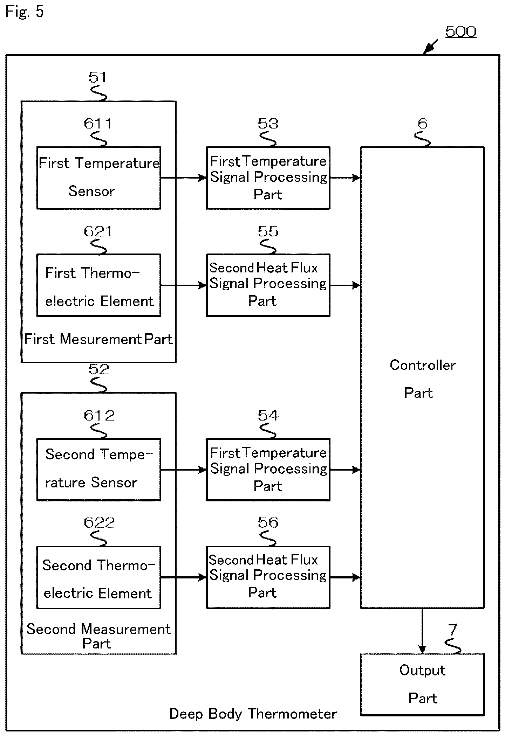

Fig. 5 shows a block diagram illustrating the entire configuration of deep body thermometer according to modified embodiment 3.

As shown in Fig. 5, the deep body thermometer 500 comprises first and second measurement parts 51 and 52, first and second temperature-signal processing parts 53 and 54, first and second heat-flux signal processing parts 55 and 56, the controller part 6 and the output part 7.

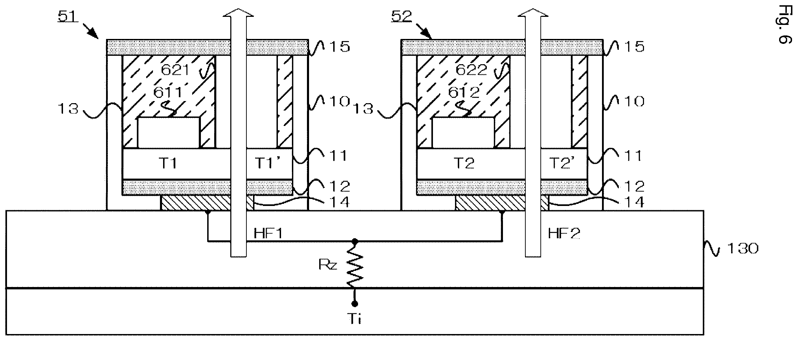

Fig. 6 shows the cross-sectional view of configuration example according to the modified embodiment 3.

As shown in Fig. 6, the substrate 11 of first measurement part 51 is arranged with providing a first temperature sensor 611 and a first thermo-electronic element 621 in close proximity on the face opposite to the measuring face such that the temperature T1 measured by the first temperature sensor 611 and the temperature T 1' at the lower face (measuring side face) of first thermo-electric element 621 becomes approximately equal. The first temperature sensor 611 is connected with the controller part 6 via. printed wirings not shown and outputs electric signals (voltage value) indicating the measured temperature T1 via. printed wirings not shown. The first thermo-electric element 621 is connected with the controller part 6 via. printed wirings not shown and outputs electric signals (voltage value) indicating the measured heat flux amount HF1 via. printed wirings not shown.

Furthermore, the substrate 11 of second measurement part 52 is arranged with providing a second temperature sensor 612 and a first thermo-electronic element 622 in close proximity on the face opposite to the measuring face side such that the temperature T2 measured by the second temperature sensor 612 and the temperature T2' at the lower face (measuring face side) of second thermo-electric element 622 becomes approximately equal. The second thermo-electric element 612 is connected with the controller part 6 via. printed wirings not shown and outputs electric signals (voltage value) indicating the measured temperature T2 via. printed wirings not shown. In this modified embodiment, since the second thermo-electric element 622 has the different thermal resistance from the first thermo-electric element 621 such that the second thermo-electric element 622 measures the heat flux HF2 different from the heat flux HF1 that has been measured by the first thermo-electric element 621. The second thermo-electric element 622 connected with the controller part 6 via. printed wirings not shown and outputs electric signals (voltage value) indicating the measured heat flux amount HF2 via. printed wirings not shown.

The heat diffusion layer 12 is disposed on the face at the measuring face side of substrate 11 and diffuses the heat entered from the heat collection plate 14; thermally transfers to the direction horizontal to the measuring face; and makes the temperature distribution of substrate 11 within the face at the measuring side even. Thereby, the heat diffusion payer 12 can make the temperature T1 measured by the first temperature sensor 611 and the temperature T 1' at the lower face (measuring fade side) of first thermo-electric element 621 approximately equal. As the result, the first temperature sensor 611 can measure the temperature at the subject side face (lower face) of first thermo-electric element 621. Furthermore, the heat diffusion layer 12 can make the temperature T2 measured by the second temperature sensor 612 and the temperature T2' at the lower side (measuring side face) of second thermo-electric element 622 even. As the result, the second temperature sensor 612 can measure the temperature at the subject side face (lower face) of second thermo-electric element 622.

The first temperature-signal processing part 53 shown in Fig. 5 amplifies the analog electric signals input from the first temperature sensor 611; then converts into the digital electric signals; and outputs them. The second temperature-signal processing part 54 amplifies the analog electric signals input from the second temperature sensor 612; then converts into the digital electric signals; and outputs them. The first heat-flux signal processing part 55 amplifies the analog electric signals input from the first thermo-electric element 621; then converts into the digital electric signals; and outputs them. The second heat-flux signal processing part 56 amplifies the analog electric signals input from the second thermo-electric element 622; then converts into the digital electric signals; and outputs them.

Then, the controller part 6 may obtain the deep body temperature Ti of subject by substituting into Equation (3) the temperature T1 ($\fallingdotseq$T1') and the heat flux amount HF1, measured by the first measurement part 51 and the temperature T2 ($\fallingdotseq$T2') and the heat flux amount HF2 measured by the second measurement part 52.

As described above, in the deep body thermometer 500 according to the present modified embodiment, the measurement part comprises the first measurement part 51 and the second measurement part 52. The first measurement part 51 comprises the first thermo-electric element 621 and the first temperature sensor 611 that measures the temperature of the subject side face of first thermo-electric element 621. Furthermore, the first measurement part 51 outputs the heat flux amount HF1 measured by the first thermo-electric element 621 and the first temperature T1 measured by the first temperature sensor 611. The second measurement part 52 comprises the second thermo-electric element 622 that has the thermal resistance value different from the first thermo-electric element 621 and the second temperature sensor 612 that measures the temperature of the subject side face of second thermo-electric element 622. Furthermore, the measurement part 52 outputs the heat flux amount HF2 measured by the second thermo-electric element 622 and the second temperature T2 measured by the second temperature sensor 612.

As described above, the deep body thermometer 500, without providing the heat flux sensor having high-cost sandwich structure, can realize the approximately same function as the heat flux sensor having the sandwich structure by using the first and the second temperature sensors 611 and 612 together with the first and the second thermo-electric elements 621 and 622 such that the deep body thermometer 500 can be manufactured in lower cost than conventional one.

Here, also in the present modified embodiment, as the measurement part 41 of modified embodiment 1, the first and the second temperature sensors 611 and 621 may be disposed at the lower face (face at the measuring face side face) of first and second thermo-electric elements 612 and 622.

In addition, as the measurement part 42 according to the modified embodiment 2, the first and the second temperature sensors 611 and 621 may be each disposed to the upper face (face opposite to the measuring face) of the heat conduction sheet 15 of first and second thermo-electric elements 612 and 622.

[Modified Embodiment 4]

Next, the modified embodiment 4 will be described, which comprises two thermo-electric elements each having different rolls such as the thermo-electric element that measures the heat flux of subject and the thermo-electric element that is used as the heat source.

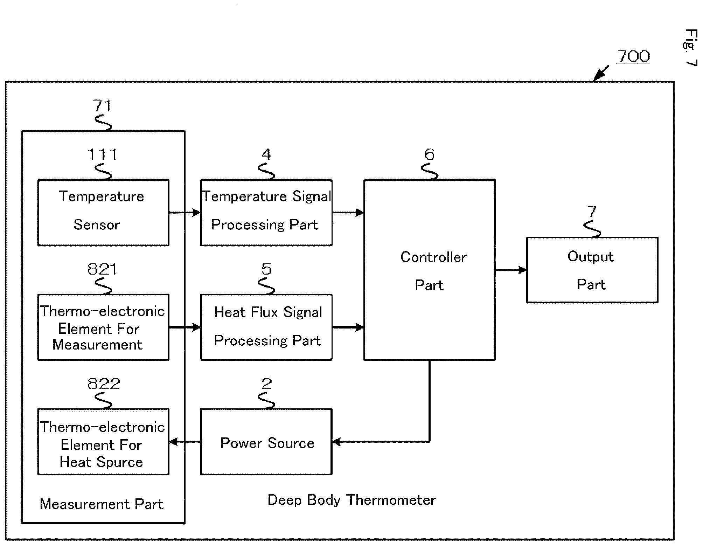

Fig. 7 shows the block diagram illustrating the entire configuration of deep body thermometer according to the modified embodiment 4.

As shown in Fig. 7, the deep body thermometer 700 comprises a measurement part 71, the power source 2, the temperature-signal processing part 4, the heat-flux signal processing part 5, the controller part 6 and the output part 7.

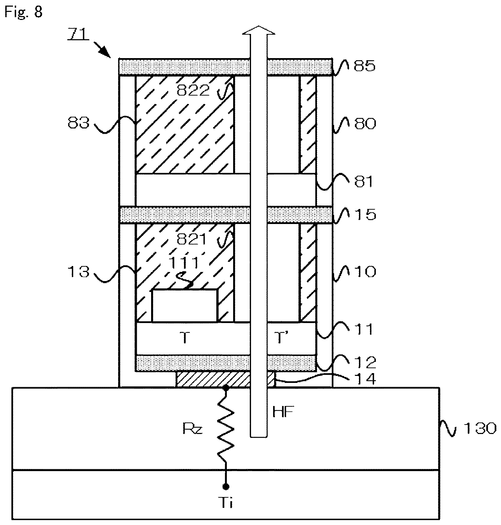

Fig. 8 shows a cross-sectional view illustrating the configuration example of measurement part according to the modified embodiment 4.

As shown in Fig. 8, the substrate 11 of measurement part 71 is arranged with disposing a temperature sensor 111 and a thermo-electric element 821 for measurements in close proximity on the face opposite to the measuring face such that the temperature T that is measured by the temperature sensor 111 and the temperature T' at the lower face (measuring face side) of thermo-electric element 821 for measurements becomes approximately equal. Furthermore, on the upper face of heat conduction sheet 15 (face opposite to the measuring face), the substrate 81 is disposed. The substrate 81 is arranged with disposing a thermo-electric element 822 for heat source on the face opposite to the measuring face at the position corresponding to the thermo-electric element 821. That is, the measurement part 71 according to the modified embodiment 4 comprises two thermo-electric elements each having different rolls such as the thermo-electric element 821 for measuring the heat flux of subject and the thermo-electric element 822 used as the heat source.

The thermo-electric element 821 for measurement is one that functions as the heat flux sensor and obtains the heat flux amount HF from deep positions such as the brain or organs of subject. The thermo-electric element 821 for measurement is electrically connected with the controller part 6 via. printed wirings not shown and outputs electric signals (voltage value) indicating the measured heat flux amount HF via. printed wirings not shown.

The thermo-electric element 822 is one that functions as the heat source, and when the electric power is supplied from the power source 2, allows to flow direct currents so as to heat or cool the body surface of subject (to change the temperature of subject's surface) together with so as to change the heat flux amount from the deep position.

The heat diffusion layer 12 is disposed on the face at the measuring face side of substrate 11 and diffuses the heat entered from the heat collection plate 14 and allows heat conduction to the direction horizontal to the measuring face such that temperature distribution within the face at the measuring surface of substrate 11 makes even. Thereby, the heat diffusion layer 12 can make the temperature T measured by the temperature sensor 111 and the temperature T' at the lower face (measuring face side) of thermo-electric element 821 for heat measurement approximately equal. As the result, the temperature sensor 111 can measure the temperature of the face at the subject's side (lower surface) of thermo-electric element 821 for measurement.

The heat insulation layer 13 is filled in a case 80 for covering the thermo-electric element 822 for heat source. In addition, to the face opposite to the measuring face of case 10, the heat conduction sheet 85 is disposed.

The controller part 6 shown in Fig. 7 first turn off the power source 2 to terminate the thermo-electric element 822. Next, the controller part 6 obtains the temperature T (≒T') indicated by the electric signal input from the temperature-signal processing part 4 and the heat flux amount HF indicated by the electric signal input from the heat-flux signal processing part 5 as the temperature T1 and the heat flux HF1 of subject at the normal condition before heating or cooling the body surface of subject.

Next, the controller part 6 turns on the power source 2 to start the thermo-electric element 822 for heat source and obtains the temperature T indicated by the electric signals input from the temperature-signal processing part 5 and the heat flux amount HF indicated by the electric signals input from the heat-flux signal processing part 4. Furthermore, the controller part 6 determines whether or not the obtained heat flux amount HF becomes constant, and if becoming constant, obtains the temperature T (≒ T') input from the temperature-signal processing unit 4 and the heat flux amount HF input from the heat-flux signal processing part 5 as the temperature T2 and the heat flux amount HF2 of subject at the equilibrium condition.

Then, the controller part 6 may obtain the deep body temperature Ti of subject by substituting into the equation (3) the temperature T1 and the heat flux amount HF1 obtained at the normal condition together with the temperature T2 and the heat flux amount HF2 obtained at the thermal equilibrium condition.

Next, the deep body temperature measuring processing executed by the deep body thermometer 700 comprising the above configuration will be described with reference to the drawings.

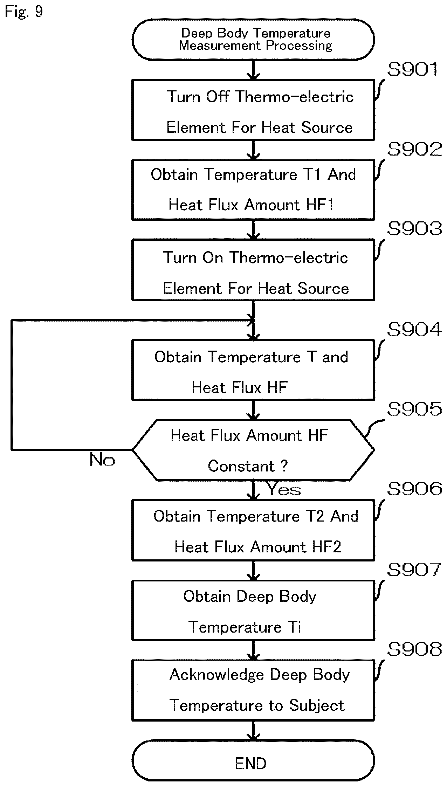

Fig. 9 shows a flowchart illustrating the detail of deep body temperature measuring processing according to the modified embodiment 4.

In the deep body temperature measuring processing shown in Fig. 9, the controller part 6 of deep body thermometer 700 first turns off the electric power 2 to terminate the thermo-electric element 822 for heat source (Step S901).

Next, the controller part 6 obtains the temperature T (≒T') indicated by the electric signal input from the temperature-signal processing part 4 and the heat flus amount HF indicated by the electric signal input from the heat-flux signal processing part 5 as the temperature T1 and the heat flux HF 1 of subject at the normal condition before heating or cooling the body surface of subject (Step S902).

Next, the controller part 6 turns on the power source 2 to start the thermo-electric element 822 for heat source (Step S903) and obtains the temperature T (≒T') indicated by the electric signals input from the temperature-signal processing part 4 and the heat flux amount HF indicated by the electric signals input from the heat-flux signal processing part 5 (Step S904).

Subsequently, the controller 6 determines whether or not the heat flux amount HF obtained in Step S904 becomes constant (Step S905), and if not becoming constant (Step S905; No), returns to Step S904 to obtain the temperature T (≒T') and the heat flux amount HF again and determines whether or not the heat flux amount HF becomes constant.

Then, the controller part 6, when the heat flux HF obtained in Step S904 becomes constant (Step S905; Yes), obtains the temperature T (≒T') indicated by the electric signal input from the temperature-signal processing part 4 and the heat flus amount HF indicated by the electric signal input from the heat-flux signal processing part 5 as the temperature T2 and the heat flux HF2 of subject at the thermal equilibrium condition (Step S906).

Subsequently, the controller part 6 obtains (measures) the deep body temperature Ti of subject by substituting into the equation (3) the temperature T1 and the heat flux amount HF1 obtained at the normal condition together with the temperature T2 and the heat flux amount HF2 obtained at the thermal equilibrium condition (Step S907).

Then, the controller part 6 displays the obtained deep body temperature in Step S907 on the display screen of output part 7 to acknowledge the deep body temperature Ti to the subject (Step S908) and terminates the deep body temperature Ti measurement processing.

As described above, in the deep body thermometer 700 according to the present modified embodiment, the measurement part 71 further comprises the thermo-electric element 822 for heat source, which is used as the heat source. The controller part 6 obtains the heat flux amount HF1 measured by the thermo-electric element 821 for measurement and the temperature T1 measured by the temperature sensor 111. Then, the controller part 6, after changing the temperature of subject's surface by supplying the electric power to the thermo-electric element 822 for heat source, obtains the heat flux HF2 measured by the thermo-electric element 821 and the temperature T2 measured by the temperature sensor 111.

As described above, the deep body thermometer 1, without providing the heat flux sensor having high-cost sandwich structure, can realize the approximately same function as the heat flux sensor having the sandwich structure by using the temperature sensor 111 and the thermo-electric element 112 such that the deep body thermometer can be manufactured in lower cost than conventional one.

Here, also in the present modified embodiment, as the measurement part 41 according to the modified embodiment 1, the temperature sensor 111 may be disposed at the lower face (face at the measuring face side) of thermo-electric element 821 for measurement. Furthermore, as the measuring part 42 according to the modified embodiment 2, the temperature sensor 111 may be disposed at the upper face (face opposite to the measuring face) of heat conduction sheet 85 placed above the thermo-electric element 821 for measurement.

Subsequently, a modified embodiment 5 will be described, which two temperature sensors and a thermo-electric element are each disposed at equal spacings.

[Modified Embodiment 5]

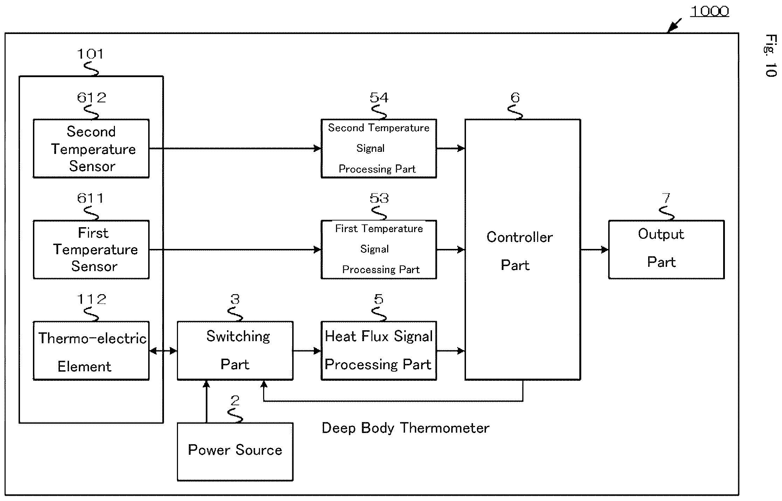

Fig. 10 shows a block diagram illustrating the entire configuration of deep body thermometer according to modified embodiment 5.

As shown in Fig. 10, the deep body thermometer 1000 comprises a measurement part 101, the power source 2, the switching part 3, first and second temperature-signal processing parts 53 and 54, the heat-flux signal processing part 5, the controller part 6, and the output part 7.

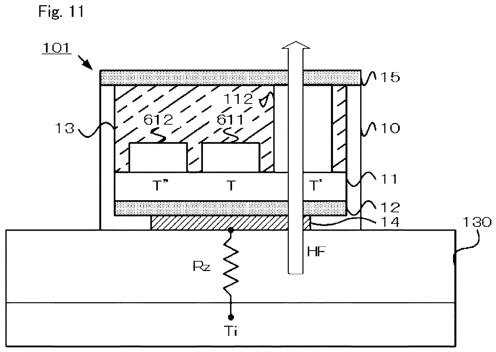

Fig. 11 shows a cross-sectional view illustrating the configuration example of measurement part according to the modified embodiment 5.

As shown in Fig. 11, the substrate 11 of measurement part 101 is arranged with disposing a second temperature sensor 612, a first temperature sensor 611 and a thermo-electric element 112 with approximately equal spacings and in close proximity on the face opposite to the measuring face such that the temperature T" measured by the second temperature sensor 612, the temperature T measured by the first temperature sensor 611 and the temperature T' at the lower face (measuring face side) of thermo-electric element 112 becomes approximately equal.

The first temperature sensor 611 is electrically connected with the controller part 6 via. printed wirings not shown and outputs electric signals (voltage value) indicating the measured temperature T via. printed wirings not shown. The second temperature sensor 612 is electrically connected with the controller part 6 via. printed wirings not shown and outputs electric signals (voltage value) indicating the measured temperature T" via. printed wirings not shown.

The heat diffusion layer 12 diffuses the heat entered from the heat collection plate 14; thermally transfers to the direction horizontal to the measuring face; and makes the temperature distribution of substrate 11 within the face at the measuring side. Thereby, the heat diffusion layer 12 can make the temperature T" measured by the second temperature sensor 612, the temperature T measured by the first temperature sensor 611 and the temperature T' at the lower face (measuring face side) of thermo-electric element 112 approximately equal. As the result, the first and second temperature sensors 611 and 612 can measure the temperature of the subject' side face (lower face) of thermo-electric element 112.

The first temperature-signal processing part 53 shown in Fig. 10 amplifies analog electric signals input from the first temperature sensor 611; then converts to digital electric signals; and outputs them. The second temperature-signal processing part 54 amplifies analog electric signals input from the second temperature sensor 612; then converts to digital electric signals; and outputs them.

The controller part 6 determines whether or not the temperature T input from the first temperature-signal processing unit 53 becomes approximately equal to the temperature T" input from the second temperature-signal processing part 54. The controller part 6, when the temperature T is approximately equal to the temperature T", determines that the temperature T' of the lower face (measuring face side) of thermo-electric element 112 is also approximately equal to the temperature T and T" and obtains the temperature T (or T") as one that indicates the temperature T' at the lower face (measuring face side) of thermo-electric element 112.

Now, when the temperature T is not approximately equal to the temperature T", since the second temperature sensor 612, the first temperature sensor 611 and the thermo-electric element 112 are disposed in the approximately equal spacings, the difference (T-T") between the temperature T measured by the first temperature sensor 611 and the temperature T" measured by the second temperature sensor 115 and the difference (T'-T) between the temperature T' at the lower face (measuring face side) of thermo-electric element 112 and the temperature T measured by the first temperature sensor 111 become approximately equal and satisfies the following equation (4).

$$T-T" = T'-T \tag{4}$$

Thus, if the temperature T is not approximately equal to the temperature T", the temperature T' at the lower face (measuring face side) of thermo-electric element 112 is represented by the following equation (5).

$$T' = 2T-T" \tag{5}$$

Therefore, the controller part 6, if the temperature T is not approximately equal to the temperature T", may obtain the temperature T' of the thermo-electric element 112 by substituting the temperature T and T" into the equation (5).

As described above, in the deep body thermometer 1000 according to the present modified embodiment, the temperature sensor comprises the first temperature sensor 611 and the second temperature sensor 612. In addition, the measurement part 101 is arranged with equipping the second temperature sensor 612, the first temperature sensor 611 and thermo-electric element 112 in approximately equal spacings. The controller part 6, when the temperature measured by the second temperature sensor 612 is approximately same with the temperature measured by the first temperature sensor 611, obtains the temperature measured by the first temperature sensor 611 as the temperature of the thermo-electric element 112 at the subject's side face. Contradictory to the above, the controller part 6, when the temperature measured by the second temperature sensor 612 is not approximately same with the temperature measured by the first temperature sensor 611, obtains the temperature of the thermo-electric element 112 at the subject's side face from the difference between the temperature measured by the second temperature sensor 612 and the temperature measured by the first temperature sensor 611 and the temperature measured by the first temperature sensor 611.

As described above, the deep body thermometer 1000 without providing the heat flux sensor having high-cost sandwich structure, can realize the approximately same function as the heat flux sensor having the sandwich structure by the first and second temperature sensors 611 and 612 as well as the thermo-electric element 112 such that the deep body thermometer 1000 can be manufactured in lower cost than conventional one. Furthermore, the deep body thermometer 1000, by arranging the second temperature sensor 612, the first temperature sensor 611 and the thermo-electric element 112 in approximately equal spacings, can measure the temperature of thermo-electric element 112 at the lower face (measuring face side), that is, the deep body temperature Ti more precisely.

Here, as the deep body thermometer 1000 according to the present modified embodiment, the configuration, to which two temperature sensors and the thermo-electric element are each disposed in the same spacings, may be applicable to the deep body thermometers 500 and 700 according to the modified embodiments 3 and 4 as well as the deep body thermometer 100 according to the present embodiment.

In the above embodiments, the object to be measured is a subject, that is, a human being; however, the present invention should not be limited thereto, the object to be measured may be animals.

In the above embodiments, the measurement part 1 has been explained as one that measures the deep body temperature by contacting with the body surface of subject. However, the present invention should not be limited thereto, one that measures the deep body temperature without contacting (contactless) with the body surface of subject may be contemplated.

In the above embodiments, the deep body thermometer 100, 500, 700 and 1000 has been described as ones that issue a predetermined alarm for the fear of heat fever when the deep body temperature of subject satisfies a predetermined condition. However, the present invention should not be limited thereto, one that issues the alarm of fear for an emergency to mind and body other than the heat fever, when the deep body temperature of subject satisfies a predetermined condition, may be contemplated; any emergency of mind and body relating to the deep body temperature may be contemplated, and for example, hypothermia, quality of sleep, basal body temperature, immunity, stress may be included.

In the above embodiments, as the internal temperature measurement device 100, 500, 700, 1000, the deep body temperature for measuring the deep body temperature Ti has been exemplarily described. However, the internal temperature measurement device according to the present invention should not be limited thereto, one that analyze electric circuits by sending pulsed signals or alternative signals may be contemplated. For example, one that measures the compositions of human body by impedance (resistance value under alternative signal) may be contemplated. Furthermore, one that performs the analysis of physical properties such as measuring water content by generating thermal pulses may be contemplated.

In the above embodiment, as the internal temperature measurement device, the deep body thermometer 100, 500, 700 and 1000, which is equipped on the body surface such as the head or the trunk of body and measures the deep body temperature Ti that is the body temperature at the deep position such as brain or organs, have been exemplarily described. However, the internal temperature measurement device according to the present invention should not be limited thereto, one that is equipped to other than the trunk of body so as to estimate (including inference etc.) the internal body temperature may be contemplated. For example, the internal body thermometer may be one that measures the internal body temperature at peripheral positions by equipping to the peripheral positions distal from the trunk of body such as the arm or the ankle etc. of subject so as to estimate (also including inference) the internal temperature at the peripheral positions may be contemplated.

In this case, the controller part 6 may estimate the deep body temperature Ti from the internal temperature of subject at the peripheral positions. Particularly, internal body temperatures and deep body temperatures of subjects have been measured beforehand to obtain correlations between them and these may be stored in the controller part 6. Then, the controller part 6 may estimate the deep body temperature Ti from measured internal body temperature of subject at the peripheral position using the correlations obtained beforehand. For example, in the case where the correlation that the deep body temperature Ti is approximately higher by 5 °C than the internal body temperature of peripheral positions has been obtained, if the measured internal body temperature of subject's peripheral position is 32 °C, 37 °C may be estimated by adding the constant of 5 °C. In addition, the controller part 6 may issue the alarm of heat fever to the subject when the predetermined condition is satisfied such as the case where the estimated deep body temperature exceeds the predetermined threshold and the like.

In the above embodiments, the program that the CPU of controller part 6 executes has been described as one stored in ROM beforehand; however, the present invention should not be limited thereto, the program for executing the above processings may be implemented to an existing and general-purpose computer for functioning as the deep body thermometer 100, 500, 700 and 1000 according to the present embodiments.

Methods for distributing such program may be optional and may include, for example, distributing by storing in a computer readable storage medium (flexible disk, CD (Compact Disc)-ROM, DVD (Digital Versatile Disc)-ROM and/or providing by allowing download of the program stored in storage on a network such as INTERNET etc.

Furthermore, when the above processing is executed by sharing between OS (Operating System) and an application program or by collaboration of OS and the application program, only the application program can be stored in the storage medium or the storage. Besides, delivery through the network by overlaying the program on carrier waves may be allowed. For example, the program may be posted on a board on the network (BBS: Bulletin Board System) and may be distributed through the network. In addition, the configuration may be allowed to start and to make the program execute likely to other application programs under the control of OS so as to allow the above processing to be able to execute.

Now, various embodiments and modifications may be made to the present invention without departing from the spirit and scope in wider meaning of the present invention. Furthermore, the above embodiments are provided merely descriptive purpose for describing one embodiment and should not limit the scope of the present invention.

Description of Signs

1, 41, 42, 71, 101 measurement part
2 power source
3 switching part
4 temperature-signal processing part
5 heat-flux signal processing part
6 controller part
7 output part
10 case
11, 81 substrate
12 heat diffusion layer
13 heat insulation layer
14 heat collection issue
15, 85 heat conduction sheet
51 first measurement part

52 second measurement part
53 first temperature-signal processing part
54 second temperature-signal processing part
55 first heat-flux signal processing part
56 second heat-flux signal processing part
100, 500, 700, 1000 deep body thermometer (internal temperature measurement device)
111 temperature sensor
112 thermo-electric element
130 subcutaneous tissue
611 first temperature sensor
612 second temperature sensor
621 first thermo-electric element
622 second thermo-electric element
821 thermo-electric element for measurement
822 thermo-electric element for heat source

**Claims**

1.  An internal temperature measurement device (100, 500, 700, 1000) comprising:

    a thermo-electric element (112, 621, 622, 821) for measuring a heat flux from an internal position of an object to be measured, a temperature sensor (111, 611, 612) for measuring temperature of a face of the thermo-electric element (112, 621, 622, 821) at a side of the object to be measured, a measurement part (1, 41, 42, 51, 52, 71, 101) for outputting a first heat flux amount measured by the thermo-electric element (112, 621, 821), a first temperature measured by the temperature sensor (111, 611) at the first heat flux amount, a second heat flux amount different from the first heat flux amount measured by the thermo-electric element (112, 622, 821) and a second temperature measured by the temperature sensor (111, 612) at the second heat flux amount; and
    a controller part (6) for obtaining the internal temperature of the object to be measured based on the first heat flux amount, the first temperature, the second heat flux amount and the second temperature input from the measurement part (1, 41, 42, 51, 52, 71, 101).

2.  The internal temperature measurement device (100, 700, 1000) of claim 1, wherein the controller part (6) obtains the first heat flux amount and the first temperature from the measurement part (1, 41, 42, 71);

    after changing temperature of a surface of the object to be measured by supplying electric power to the thermo-electric element (112, 822), obtains the second heat flux amount and the second temperature from the measurement part (1, 41, 42, 71); and
    obtains an internal temperature of the object to be measured based on the first heat flux amount, the first temperature, the second heat flux amount and the second temperature.

3.  The internal temperature measurement device (700) of claim 1, wherein the measurement part (71) further comprises a thermo-electric element (822) for heat source used as a heat source, and
    wherein

    the controller part (6) obtains the first heat flux amount measured by the thermo-electric element (821) and the first temperature measured by the temperature sensor (111), and
    after changing temperature of a surface of the object to be measured by supplying electric power to the thermo-electric element (822), obtains the second heat flux amount measured by the thermo-electric element (821) and the second temperature measured from the temperature sensor (111).

4.  The internal temperature measurement device (100, 700, 1000) of claim 2, wherein the controller part (6), after supplying electric power to the thermo-electric element (112, 822) for a predetermined duration, determines whether or not the heat flux amount input from the measurement part (1, 41, 42, 71) becomes constant, and
    when determined that the heat flux amount becomes constant, obtains the heat flux amount and the temperature input from the measurement part (1, 41, 42, 71) as the second heat flux amount and the second temperature.

5.  The internal temperature measurement device (1000) of claim 1, wherein the temperature sensor (611, 612) comprises a first temperature sensor (611) and a second temperature sensor (612); and wherein

the measurement part (101) is arranged with disposing the second temperature sensor (612), the first temperature sensor (611) and the thermo-electric element (112) in approximately equal spacings;

the controller part (6), when the temperature measured by the second temperature sensor (612) and the temperature measured by the first temperature sensor (611) are approximately same, obtains temperature obtained by the first temperature sensor (611) as temperature of a face of the thermo-electric element (112) at a side of the object to be measured; and

when the temperature measured by the second temperature sensor (612) and the temperature measured by the first temperature sensor (611) are not approximately same, obtains temperature of a face of the thermo-electric element (112) at a side of the object to be measured from a difference between temperature measured by the second temperature sensor (612) and temperature measured by the first temperature sensor (611) and temperature measured by the first temperature sensor (611).

6. The internal temperature measurement device (500) of claim 1, wherein the measurement part (51, 52) comprises a first measurement part (51) and a second measurement part (52), and wherein

the first measurement part (51) comprises a first thermo-electric element (621) and a first temperature sensor (611) for measuring temperature of a face of the thermo-electric element (621) at a side of the object to be measured and outputs the first heat flux amount measured by the first thermo-electric element (621) and the first temperature measured by the first temperature sensor (611), and

the second measurement part (52) comprises a second thermo-electric element (622) having a heat resistance value different from the first thermo-electric element (621) and a second temperature sensor (612) for measuring temperature of a face of the thermo-electric element (622) at a side of the object to be measured and outputs the second heat flux amount measured by the second thermo-electric element (622) and the second temperature measured by the second temperature sensor (612).

7. An internal temperature measurement method comprising: by a measurement part (1, 41, 42, 51, 52, 71, 101) comprising a thermo-electric element (112, 621, 622, 821) for measuring a heat flux amount from an internal position of an object to be measured and a temperature sensor (111, 611, 612) for measuring temperature of a face of the thermo-electric element (112, 621, 821) at a side of the object to be measured;

outputting a first heat flux amount measured by the thermo-electric element (112, 621, 821), a first temperature measured by the temperature sensor (111, 611) at the first heat flux amount, a second heat flux amount different from the first heat flux amount measured by the thermo-electric element (112, 622, 821) and a second temperature measured by the temperature sensor (111, 612) at the second heat flux amount; and

obtaining an internal temperature of the object to be measured based on the first heat flux amount, the first temperature, the second heat flux amount and the second temperature input from the measurement part (1, 41, 42, 51, 52, 71, 101).

8. A program for making a computer of an internal temperature measurement device (100, 500, 700, 1000) comprising a measurement part (1, 41, 42, 51, 52, 71, 101) comprising a thermo-electric element (112, 621, 622, 821) for measuring a heat flux amount from an internal position of an object to be measured and a temperature sensor (111, 611, 612) for measuring temperature of a face of the thermo-electric element (112, 621, 821) at a side of the object to be measured and outputting a first heat flux amount measured by the thermo-electric element (112, 621, 821), a first temperature measured by the temperature sensor (111, 611) at the first heat flux amount, a second heat flux amount different from the first heat flux amount measured by the thermo-electric element (112, 622, 821) and a second temperature measured by the temperature sensor (111, 612) at the second heat flux amount to execute;

obtaining an internal temperature of the object to be measured based on the first heat flux amount, the first temperature, the second heat flux amount and the second temperature input from the measurement part (1, 41, 42, 51, 52, 71, 101).

EP 4 483 789 A1

Fig. 1

100

Deep Body Thermometer

Measurement Part

1

111
Temperature Sensor

112
Thermo-electric Element

3
Switching Part

2
Power Source

4
Temperature Signal Processing Part

5
Heat Flux Signal Processing Part

6
Controller Part

7
Output Part

EP 4 483 789 A1

Fig. 2

17

Fig. 3

Deep Body Temperature
Measurement Processing

S301
Function Thermo-electric
Element As Heat Flux Sensor

S302
Obtain Temperature T1
And Heat Flux Amount HF1

S303
Function Thermo-electric
Element As Heat Source

S304
Predetermined Duration
Passed ?
No
Yes

S305
Function Thermo-electric
Element As Heat Flux Sensor

S306
Obtain Temperature T And
Heat Flux Amount T

S307
Heat Flux Amount
Constant ?
No
Yes

S308
Obtain As Temperature T2
And Heat Flux HF2

S309
Obtain Deep Body
Temperature Ti

S310
Acknowledge Deep Body
Temperature Ti To Subject

END

Fig. 4

(a)

(b)

Fig. 5

Fig. 6

Fig. 7

700

Deep Body Thermometer

7 — Output Part

6 — Controller Part

4 — Temperature Signal Processing Part

5 — Heat Flux Signal Processing Part

2 — Power Source

71 — Measurement Part

111 — Temperature Sensor

821 — Thermo-electronic Element For Measurement

822 — Thermo-electronic Element For Heat Spurce

Fig. 8

Fig. 9

```
        ┌─────────────────────────────┐
        │   Deep Body Temperature     │
        │   Measurement Processing    │
        └─────────────┬───────────────┘
                      ↓                      S901
        ┌─────────────────────────────┐
        │   Turn Off Thermo-electric  │
        │   Element For Heat Source   │
        └─────────────┬───────────────┘
                      ↓                      S902
        ┌─────────────────────────────┐
        │   Obtain Temperature T1 And │
        │   Heat Flux Amount HF1      │
        └─────────────┬───────────────┘
                      ↓                      S903
        ┌─────────────────────────────┐
        │   Turn On Thermo-electric   │
        │   Element For Heat Source   │
        └─────────────┬───────────────┘
                      ↓                      S904
        ┌─────────────────────────────┐
        │   Obtain Temperature T and  │
        │   Heat Flux HF              │
        └─────────────┬───────────────┘
                      ↓                      S905
              ╱───────────────────╲
         No  │  Heat Flux Amount HF │
        ─────┤     Constant ?       │
             ╲───────────────────╱
                      ↓ Yes               S906
        ┌─────────────────────────────┐
        │   Obtain Temperature T2 And │
        │   Heat Flux Amount HF2      │
        └─────────────┬───────────────┘
                      ↓                      S907
        ┌─────────────────────────────┐
        │   Obtain Deep Body          │
        │   Temperature Ti            │
        └─────────────┬───────────────┘
                      ↓                      S908
        ┌─────────────────────────────┐
        │   Acknowledge Deep Body     │
        │   Temperature to Subject    │
        └─────────────┬───────────────┘
                      ↓
        ┌─────────────────────────────┐
        │            END              │
        └─────────────────────────────┘
```

Fig. 10

Fig. 11

101

612  112  611

13

T''  T  T'

15
10
11
12
14
130

HF

Rz

Ti

## INTERNATIONAL SEARCH REPORT

| International application No. |
| --- |
| **PCT/JP2022/007428** |

| A. | CLASSIFICATION OF SUBJECT MATTER |
| --- | --- |

*A61B 5/01*(2006.01)i
FI:   A61B5/01 100

According to International Patent Classification (IPC) or to both national classification and IPC

| B. | FIELDS SEARCHED |
| --- | --- |

Minimum documentation searched (classification system followed by classification symbols)

A61B5/01

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2020-176934 A (NIPPON TELEGRAPH AND TELEPHONE CORP.) 29 October 2020 (2020-10-29) | 1, 6-8 |
| | paragraphs [0001]-[0059], fig. 1-8 | |
| A | paragraphs [0001]-[0059], fig. 1-8 | 2-5 |
| Y | JP 2018-44804 A (FUJIKURA LTD.) 22 March 2018 (2018-03-22) | 1, 6-8 |
| | paragraphs [0010], [0052]-[0054], fig. 3-5 | |
| A | JP 2019-516960 A (KONINKLIJKE PHILIPS N.V.) 20 June 2019 (2019-06-20) | 1-8 |
| | paragraphs [0027]-[0037], fig. 1 | |
| A | WO 2020/184511 A1 (BIODATA BANK, INC.) 17 September 2020 (2020-09-17) | 1-8 |
| | paragraphs [0023]-[0061], fig. 1-3 | |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **13 April 2022** | **26 April 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2022/007428**

| Patent document cited in search report | | | Publication date (day/month/year) | Patent family member(s) | | | Publication date (day/month/year) |
|---|---|---|---|---|---|---|---|
| JP | 2020-176934 | A | 29 October 2020 | WO | 2020/213437 | A1 | |
| | | | | paragraphs [0001]-[0059], fig. 1-8 | | | |
| JP | 2018-44804 | A | 22 March 2018 | (Family: none) | | | |
| JP | 2019-516960 | A | 20 June 2019 | US | 2019/0142280 | A1 | |
| | | | | paragraphs [0031]-[0041], fig. 1 | | | |
| | | | | WO | 2017/198788 | A1 | |
| | | | | EP | 3457922 | A1 | |
| WO | 2020/184511 | A1 | 17 September 2020 | JP | 6755034 | B1 | |
| | | | | paragraphs [0023]-[0061], fig. 1-3 | | | |

Form PCT/ISA/210 (patent family annex) (January 2015)

**REFERENCES CITED IN THE DESCRIPTION**

**Patent documents cited in the description**

- JP 2007212407 A

- WO 2020184511 A